# EUROPEAN PATENT SPECIFICATION

(11) **EP 4 611 641 B1**
(45) Date of publication and mention of the grant of the patent: **03.12.2025**
(21) Application number: 24801923.4
(22) Date of filing: 11.11.2024
(51) Int. Cl.: A61B 6/06

(54) **A NOVEL AND COST-EFFECTIVE WAY TO MEASURE COLLIMATOR ROTATIONAL ANGLE IN ALL THREE AXIS**
NEUARTIGE UND KOSTENEFFEKTIVE ART UND WEISE ZUR MESSUNG DES KOLLIMATORDREHWINKELS IN ALLEN DREI ACHSEN
NOUVELLE MANIÈRE ÉCONOMIQUE DE MESURER UN ANGLE DE ROTATION DE COLLIMATEUR DANS TOUS LES TROIS AXES

(30) Priority: 20.11.2023 EP 23210942
(43) Date of publication of application: 10.09.2025
(73) Proprietor: Koninklijke Philips N.V., 5656 AG Eindhoven (NL)
(72) Inventor: DIXIT, Deepak Dilipsingh, 5656 AG Eindhoven (NL); KUMAR, Rajneesh, 5656 AG Eindhoven (NL); SAMANTA, Sohham, 5656 AG Eindhoven (NL); SHRIPAD, Anilkumar, 5656 AG Eindhoven (NL)
(74) Representative: Philips Intellectual Property & Standards
(86) International application number: PCT/EP2024/081798
(87) International publication number: WO 2025/108740

(56) References cited:
- CN-U- 205 072 882
- JP-A- 2019 010 443
- JP-A- S58 169 076
- US-A1- 2006 126 791
- US-A1- 2016 220 223

## Description

### FIELD OF THE INVENTION

The present invention relates to the field of radiation based imaging. In particular the invention relates to the field of X-ray collimation, in particular to a rotational angle measurement device, to a rotational angle measurement system, to a rotational angle measurement method, to a computer program element, and to a computer-readable storage medium.

### BACKGROUND OF THE INVENTION

Collimator is a major subcomponent in an X-ray system. The collimator adjusts the X-ray field to the limit required for actual exposure. It also enables the user to do this adjustment. Collimator provides light simulation of the X-ray field. In clinical workflow scenario user needs to rotate the collimator based on patient anatomy, while doing collimator rotation angular measurement of rotation is critical for getting correct On-screen Amplimat chamber Visualization.

Exemplary rotational angle measurement devices for measuring a rotational angle of a rotatable X-ray collimator are disclosed in US 2006/126791 A1, CN 205 072 882 U, US 2016/220223 A1, JP S58 169076 A and JP 2019 010443 A.

### SUMMARY OF THE INVENTION

There may therefore be a need for a rotational angle measurement functionality.
The object of the present invention is solved by the subject matter of the independent claims where further embodiments are incorporated in the dependent claims. It is understood that the following aspect of the invention equally applies to the rotational angle measurement device, to the rotational angle measurement system, to the rotational angle measurement method, to the computer program element, and to the computer-readable storage medium. Accordingly, any feature, function, step and/or element described in the following with reference to one aspect of the present disclosure equally applies to any other aspect of the present disclosure.

According to a first aspect of the present invention, there is provided a rotational angle measurement device for measuring a rotational angle of a rotatable X-ray collimator. The rotational angle measurement device comprises a resistive strip and a plunger. The resistive strip comprises an elongated tape that is attached to a first portion of the rotatable X-ray collimator. A longitudinal extent of the elongated tape is arranged to be curved about the rotation axis of the rotatable X-ray collimator. The plunger is attached to a second portion of the X-ray collimator. The first portion and the second portion are arranged to rotate relative to each other during a rotation of the X-ray collimator. The plunger is arranged to be in contact with the elongated strip such that a rotation of the rotatable X-ray collimator moves the elongated strip relative to the plunger to produce an electrical signal indicative of a rotational angle of the rotatable X-ray collimator.

Accordingly, the present disclosure proposes a rotational angle measurement device that comprises a plunger and a resistive strip that are arranged on different parts, i.e., first portion and second portion, of the rotatable X-ray collimator. The first portion and second portion are arranged to rotate relative to each other during a rotation of the X-ray collimator. For example, the first portion is moved, i.e. rotated, during the rotation of the rotatable X-ray collimator. The second portion is not moved, i.e., remains at its position. By pressing the plunger on the resistive strip, the resistive strip produces an electrical output, e.g., voltage output. According to the rotational position of the plunger, the resistive strip outputs results into the a corresponding electrical signal indicative of the rotational angle of the rotatable X-ray collimator. The proposed rotational angle measurement device may be in combination with any X-ray system where an automatic collimation with rotational angle measurement feature is required. The proposed rotational angle measurement device may reduce the risk of wrong amplimat chamber position which may result into additional dose to patient. The proposed rotational angle measurement device may also help to improve optimal collimation and to reduce unnecessary radiation area.

This will be described in detail hereinafter and in particular with respect to the example shown in Figs. 3A, 3B, 4A, and 4B. An example of the plunger is shown in FIG. 5A, and an example of the resistive strip is shown in Fig. 5B.

According to an exemplary embodiment of the first aspect of the present invention, the plunger comprises one of a spring-loaded plunger, and a pressure-loaded plunger.

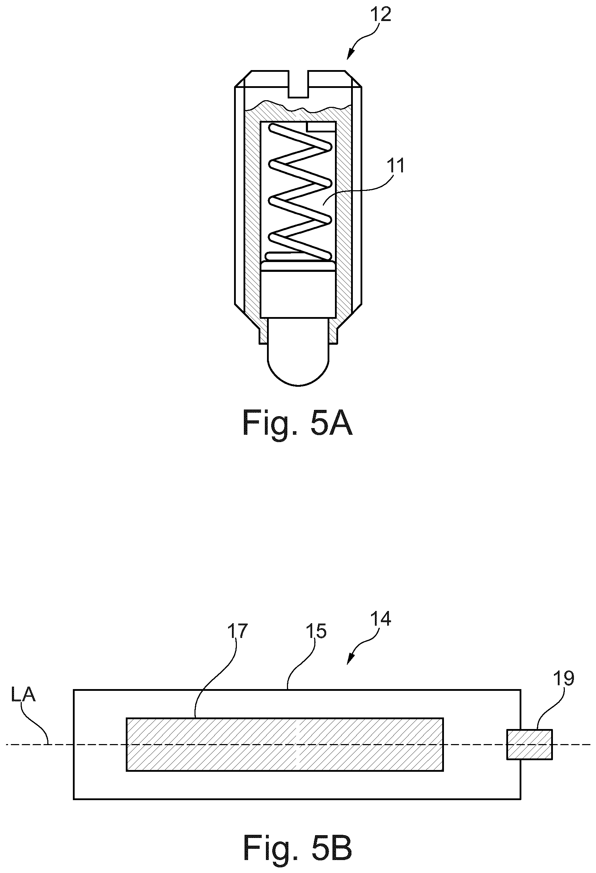

This will be described in detail hereinafter and in particular with respect to the example shown in Fig. 5A.

According to an exemplary embodiment of the first aspect of the present invention, the second portion comprises a source alignment flange that is arranged to couple the X-ray collimator to the X-ray source..

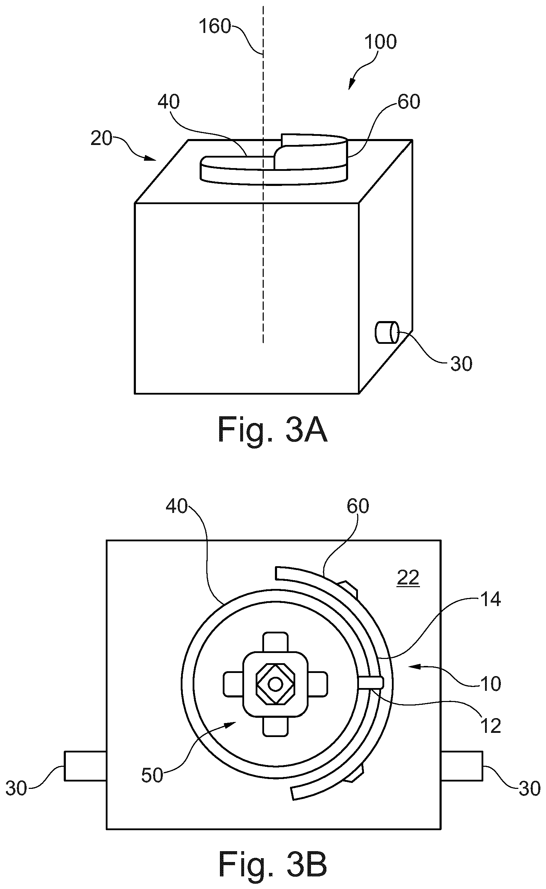

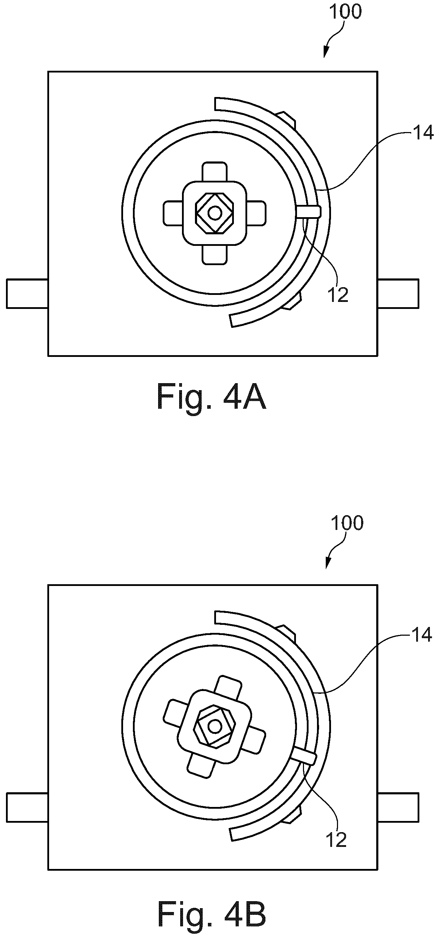

This will be described in detail hereinafter and in particular with respect to the examples shown in Figs. 3A, 3B, 4A, and 4B.

According to an exemplary embodiment of the first aspect of the present invention, the first portion comprises a strip mounting support that is arranged opposite to the source alignment flange.

This will be described in detail hereinafter and in particular with respect to the examples shown in Figs. 3A, 3B, 4A, and 4B.

According to a second aspect of the present invention, there is provided a rotational angle measurement system that comprises the rotational angle measurement device according to the first aspect and any associated example, and a processing device configured to determine a rotational angle of a rotatable X-ray collimator based on an electrical signal produced by the rotational angle measurement device.

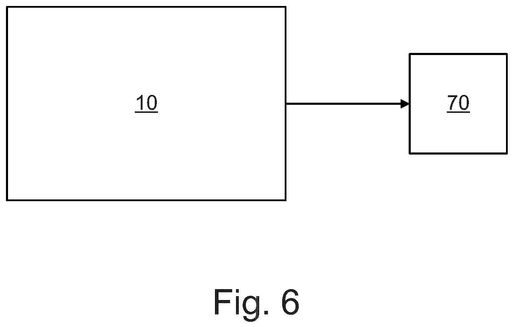

This will be described in detail hereinafter and in particular with respect to the example shown in Fig. 6.

According to an exemplary embodiment of the second aspect of the present invention, the processing device is further configured to perform a calibration of the rotational angle measurement device.

Accordingly, the correct degree of collimator rotation feedback may be achieved. This will be described in detail hereinafter and in particular with respect to the example shown in Fig. 6.

According to an exemplary embodiment of the second aspect of the present invention, the processing device is configured to perform a multi-point calibration of the rotational angle measurement device.

The multi-point calibration may comprise a two-point calibration, a three-point calibration, a fourth-point calibration, etc.

According to an exemplary embodiment of the second aspect of the present invention, the multi-point calibration comprises a seven-point calibration, wherein the seven-point calibration comprises the following angular steps: -45°, -30°, -15°, 0°, 15° , 30°, and 45°.

According to a third aspect of the present invention, there is provided an X-ray collimator that comprises the rotational angle measurement device according to the first aspect and any associated example or the rotational angle measurement system according to the second aspect and any associated example.

According to a fourth aspect of the present invention, there is provided a rotational angle measurement method for measuring a rotational angle of a rotatable X-ray collimator. The method comprises the following steps:
- controlling the rotatable X-ray collimator to adjust a rotational position;
- obtaining an electrical signal from a rotational angle measurement device according to the first aspect and any associated example, and
- determining a rotational angle of the rotatable X-ray collimator based on the electrical signal.

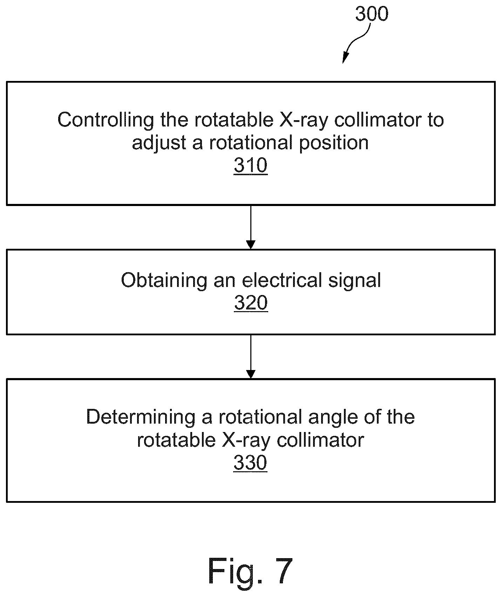

This will be described in detail hereinafter and in particular with respect to the flow chart shown in Fig. 7.

According to an exemplary embodiment of the fourth aspect of the present invention, the method further comprises the step of performing a calibration of the rotational angle measurement device.

According to an exemplary embodiment of the fourth aspect of the present invention, the step of performing a calibration of the rotational angle measurement device comprises: performing a multi-point calibration of the rotational angle measurement device.

According to an exemplary embodiment of the fourth aspect of the present invention, the multi-point calibration comprises a seven-point calibration, wherein the seven-point calibration comprises the following angular steps: -45°, -30°, -15°, 0°, 15° , 30°, and 45°.

According to another aspect of the present invention, there is provided a computer program product comprising instructions which, when the program is executed by a processing device of a rotational angle measurement system according to the second aspect of the present invention, cause the processing device to carry out the steps of the method according to the fourth aspect and any associated example. The computer program may comprise instructions, which, when the program is executed by a computer, cause the computer to carry out the method of any one of the embodiments as described herein above. The computer program element might therefore be stored on a computer unit, which might also be part of an embodiment of the present invention. This computing unit may be adapted to perform or induce a performing of the steps of the method described above. The computing unit can be adapted to operate automatically and/or to execute the orders of a user. A computer program may be loaded into a working memory of a data processor. The data processor may thus be equipped to carry out the method of the invention. This exemplary embodiment of the invention covers both, a computer program that right from the beginning uses the invention and a computer program that by means of an up-date turns an existing program into a program that uses the invention. Further on, the computer program element might be able to provide all necessary steps to fulfill the procedure of an exemplary embodiment of the method as described above.

According to a further aspect of the present invention, there is provided a computer-readable storage medium having stored thereon the computer program product. A computer program may be stored and/ or distributed on a suitable medium, such as an optical storage medium or a solid-state medium supplied together with or as part of other hardware, but may also be distributed in other forms, such as via the internet or other wired or wireless telecommunication systems. However, the computer program may also be presented over a network like the World Wide Web and can be downloaded into the working memory of a data processor from such a network. The computer-readable medium may comprise instructions which, when executed by a computer, cause the computer to carry out the method of any one of the embodiments as described herein.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

### BRIEF DESCRIPTION OF THE DRAWINGS

The aspects defined above and further aspects of the present invention are apparent from the examples of embodiment to be described hereinafter and are explained with reference to the examples of embodiment. The invention will be described in more detail hereinafter with reference to examples of embodiment but to which the invention is not limited.

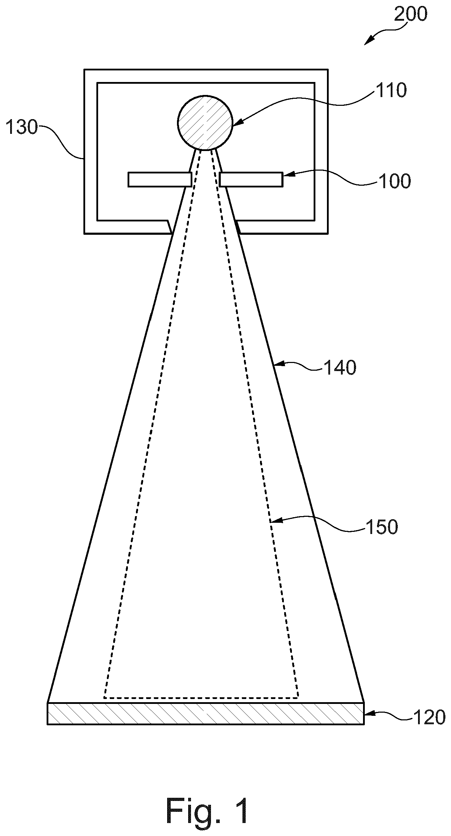

Fig. 1 illustrates an example of an X-ray collimator in an X-ray imager.

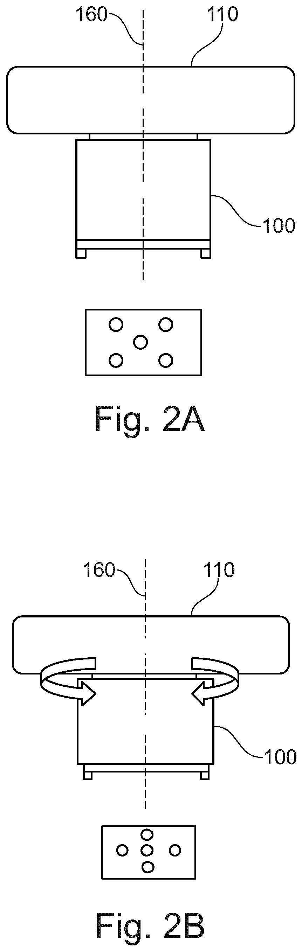

Fig. 2A illustrates the X-ray collimator at a first angular position.
Fig. 2B illustrates the X-ray collimator at a second angular position.
Fig. 3A illustrates a perspective view of the X-ray collimator.
Fig. 3B illustrates a top view of the X-ray collimator.
Fig. 4A illustrates the X-ray collimator at a first angular position.
Fig. 4B illustrates the X-ray collimator at a second angular position.
Fig. 5A illustrates an example of a plunger.
Fig. 5B illustrates an example of a resistive strip.
Fig. 6 illustrates an example of a rotational angle measurement system.
Fig. 7 illustrates a flow chart illustrating a rotational angle measurement method.

### DETAILED DESCRIPTION OF EMBODIMENTS

X-ray collimator is a major subcomponent in an X-ray system. Fig. 1 illustrates an example of an X-ray collimator 100 in an X-ray imager 200. The X-ray imager 200 comprises an X-ray source 110 and an X-ray detector 120. The X-ray source 110 and the X-ray collimator 100 are arranged in a housing 130. As shown in Fig. 1, prior to collimator interaction, an X -ray beam 140 emanating from the X-ray source 110 is a divergent beam so in absence of the X-ray collimator 100 the cross-sectional dimensions of the X-ray beam 140 when reaching the X-ray detector 120 would be much larger than the area of a desired region of interest (ROI), such as patient's lungs in a "chest X-ray". The purpose of the X-ray collimator 100 is to restrict dimensions of the cross section of the beam to create a field of view 150 that matches in size and shape the beam's cross section to the patient's ROI.

For many examinations like chest lateral and orthopedic examinations, it is often necessary to rotate the collimator for reducing the unnecessarily irradiated area. This may be done by the medical personnel by manually rotating the collimator. Between the X-ray detector 120 and the object under examination, a dose measuring device may be positioned which may be an arrangement of a plurality of measurement fields, for example five measurement fields. An example of the dose measuring device is an ionization chamber called amplimat chamber. A human operator may, before starting the irradiation with X-rays, select some (or all) of the measurement fields. Only this selected group of measurement fields will then be used during a subsequent irradiation of the object under examination with X-rays for measuring a radiation dosage during the actual irradiation with X-rays. Collimator rotational position input is crucial for proper display of On-screen Amplimat chamber Visualization. Fig. 2A illustrates an X-ray collimator 100 at an angular position of 0 degree which is in a correct amplimat chamber position. In the illustrated position, it is possible for measuring a correct radiation dosage during the actual irradiation with X-rays. However, if the X-ray collimator is at some angular position other than 0 degree and the system has no input about this, the system may control all its functionality wherever applicable by assuming collimator is at 0 degree. For example, Fig. 2B illustrates that the X-ray collimator 100 is rotated about a rotation axis 160 with 45 degrees. This may result in a wrong amplimat chamber position, and therefore an incorrect radiation dosage measurement, thereby causing additional dose to the patient under examination.

In order to address the above issues, the present disclosure proposes a rotational angle measurement device to perform collimator rotational angle measurement functionality. An exemplary arrangement of the rotational angle measurement device on the X-ray collimator is shown in Figs. 3A and 3B. As shown in both figures, the rotational angle measurement device 10 comprises a resistive strip 12 and a plunger 14.

Fig. 3A illustrates a perspective view of the X-ray collimator 100. The X-ray collimator 100 includes a collimator assembly 20 and one or more adjustment knobs 30 to adjust shutters of the collimator assembly 20. Fig. 3B illustrates a top view of the collimator assembly 20. In the illustrated example, the collimator assembly 20 includes a base 22 with an opening, a source alignment flange 40 that is used to couple the X-ray collimator to the X-ray source, e.g., X-ray tube or tube assembly, and shutters 50 to variably block X-ray radiation passing through the opening. In the illustrated example, the source alignment flange 40 is shown as a protrusion and a ring. In some other examples, the source alignment flange 40 may have another shape that may mate or coupled to the X-ray source 110. In the illustrated example, a strip mounting support 60 is provided and arranged on the base 22 of the collimator assembly 20. Although the stripe mounting support 60 is shown as a protrusion and a semi-circle, it will be appreciated that the source alignment flange 60 may have another shape. The stripe mounting support 60 is arranged opposite to the source alignment flange 40. The inner surface of the stripe mounting support 60 faces the source alignment flange 40, and is curved about the rotation axis 160 of the rotatable X-ray collimator 100.

The X-ray collimator 100 may be rotated clockwise or counterclockwise. During the rotation of the X-ray collimator, the source alignment flange 40 and the strip mounting support 60 rotate relative to each other. For example, Fig. 4A illustrates that the X-ray collimator 100 is arranged in a first rotated position. In the illustrated first rotated position, the plunger 12 is in contact with a middle point of the elongated tape of the resistive strip 14. This angular position may also be defined as angular position of 0 degree. Fig. 4B illustrates that the X-ray collimator 100 is moved to a second rotated position. As can be seen in Fig. 4B, only the plunger 12, which is attached to source alignment flange 40, is moved, i.e. rotated. The strip mounting support 60 is not moved, i.e., remains at its position. Accordingly, during the rotation of the X-ray collimator 100, the source alignment flange 40 and the strip mounting support 60 rotate relative to each other. This relative movement results in a change of the rotational position of the plunger 12 on the resistive strip 14. The rotational angle measurement device 10 then outputs results into an electrical signal, e.g., an analog voltage, which is indicative of a rotational angle of the rotatable X-ray collimator.

Fig. 5A illustrates an example of the plunger 12. In this illustrated example, the plunger 12 is a spring-loaded plunger that comprises a spring 11. However, it will be appreciated that the plunger 12 may be any type, e.g., a pressure-loaded plunger. The plunger 12 is a non-conductive mechanism. By pressing the plunger 12 onto the resistive strip 14, the resistive strip 14 produces the desired electrical output.

Fig. 5B illustrates an example of the resistive strip 14. In the illustrated example, the resistive strip 14 comprises an elongated tape 15 having an elongated extent LA. The resistive strip 14 comprises an active region 17 made of a resistive material. The resistive strip 14 further comprises an electrical channel 19, which is configured to provide an electrical output. As an example, the electrical channel 19 may include two resistive output channels and an electrical collector channel. These two resistive circuits may be separated by spacer adhesive build-up and contact between these two circuits occur by pressure from the plunger on the top circuit, pushing down until the top circuit connects with the bottom circuit and as a result it creates a potentiometric output, in form of voltage divider.

To measure the rotation angle of the X-ray collimator, the resistive strip 14 is attached to the inner surface of the strip mounting support 60, and the longitudinal extent LA of the elongated tape is arranged to be curved about the rotation axis of the rotatable X-ray collimator 100. Additionally, the plunger 12 is arranged to be in contact with the elongated strip such that a rotation of the rotatable X-ray collimator moves the elongated strip relative to the plunger to produce an electrical signal indicative of a rotational angle of the rotatable X-ray collimator. According to the rotational position of the plunger, the resistive strip 14 outputs results into the analog voltage as output of the above-mentioned voltage divider. This analog voltage signal is converted into the digital values by analog to digital converter located e.g., collimator microcontroller. From this digital voltage value, equivalent collimator rotation angle is calculated e.g., in 0.1 degree step size and communicated to system software.

Fig. 6 illustrates a rotational angle measurement system. The rotational angle measurement system comprises a rotational angle measurement device 10 as described above and a processing device 70 configured to obtain an electrical signal from the rotational angle measurement device and to determine a rotational angle of the rotatable X-ray collimator based on the electrical signal.

A "processor" is one example of the processing device 70, which employs one or more microprocessors that may be programmed using software (e.g., microcode) to perform various functions described herein. The processing device may be implemented with or without employing a processor, and also may be implemented as a combination of dedicated hardware to perform some functions and a processor (e.g., one or more programmed microprocessors and associated circuitry) to perform other functions. Examples of apparatus components that may be employed in various embodiments of the present disclosure include, but are not limited to, conventional microprocessors, application specific integrated circuits (ASICs), and field-programmable gate arrays (FPGAs). In various implementations, a processor may be associated with one or more storage media (generically referred to herein as "memory," e.g., volatile and non-volatile computer memory). In some implementations, the storage media may be encoded with one or more programs that, when executed on one or more processors and/or controllers, perform at least some of the functions described herein. Various storage media may be fixed within the computing device or may be transportable, such that the one or more programs stored thereon can be loaded into the computing device so as to implement various aspects of the present disclosure described herein. The terms "program" or "computer program" are used herein in a generic sense to refer to any type of computer code (e.g., software or microcode) that can be employed to program one or more processors. As an example, the processing unit 70 or some of its components may reside in an operator console running as software routines. The components may be programmed in a suitable scientific computing platform such as Matlab^{®} or Simulink^{®} and then translated into C++ or C routines maintained in a library and linked when called on by the operator console.

In order to achieve the correct degree of collimator rotation feedback, the processing device 70 may be further configured to perform a calibration of the rotational angle measurement device, such as a multi-point calibration of the rotational angle measurement device. As an example, to achieve +/- 0.5 degree rotational angle accuracy, the resistive strip 14 which is installed on the collimator housing may be calibrated in seven steps, such as -45°,-30°,-15°, 0°, 15° , 30° , 45°. At each of these angular steps, corresponding digital value may be stored in collimator microcontroller and the result is stored in lookup table. This lookup table helps in interpolating in between angular values with required accuracy.

In Fig. 7 there is shown a flow chart illustrating a rotational angle measurement method 300 for measuring a rotational angle of a rotatable X-ray collimator. At block 310, the method 300 comprises the step of controlling the rotatable X-ray collimator to adjust a rotational position. At block 320, the method 300 comprises the step of obtaining an electrical signal from a rotational angle measurement device as described herein. At block 330, the method 300 further comprises the step of determining a rotational angle of the rotatable X-ray collimator based on the electrical signal.

In some implementations, the method 300 may further comprise the step of performing a calibration of the rotational angle measurement device, such as a multi-point calibration of the rotational angle measurement device. For example, the multi-point calibration comprises a seven-point calibration, wherein the seven-point calibration comprises the following angular steps: -45°, -30°, -15°, 0°, 15° , 30°, and 45°.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 10: rotational angle measurement device
- 11: spring
- 12: plunger
- 14: resistive strip
- 15: elongated tape
- 17: active region
- 19: electrical channel
- 20: collimator assembly
- 30: adjustment knob(s)
- 40: source alignment flange
- 50: shutters
- 60: strip mounting support
- 70: processing device
- 100: X-ray collimator
- 110: X-ray source
- 120: X-ray detector
- 130: housing
- 140: X-ray beam
- 150: field of view
- 160: rotation axis
- 200: X-ray imager
- 300: rotational angle measurement method
- LA: elongated extent

## Claims

1. A rotational angle measurement device (10) for measuring a rotational angle of a rotatable X-ray collimator, the device comprising:
- a plunger (12); and
- a resistive strip (14);
wherein the resistive strip comprises an elongated tape that is attached to a first portion of the rotatable X-ray collimator, and an longitudinal extent of the elongated tape is arranged to be curved about the rotation axis of the rotatable X-ray collimator;
wherein the plunger is attached to a second portion of the X-ray collimator, wherein the first portion and the second portion are arranged to rotate relative to each other during a rotation of the X-ray collimator; and
wherein the plunger is arranged to be in contact with the elongated strip such that a rotation of the rotatable X-ray collimator moves the elongated strip relative to the plunger to produce an electrical signal indicative of a rotational angle of the rotatable X-ray collimator.

2. The rotational angle measurement device according to claim 1,
wherein the plunger comprises one of:
- a spring-loaded plunger; and
- a pressure-loaded plunger.

3. The rotational angle measurement device according to claim 1 or 2,
wherein the second portion comprises a source alignment flange (40) that is arranged to couple the X-ray collimator to the X-ray source.

4. The rotational angle measurement device according to claim 3,
wherein the first portion comprises a strip mounting support (60) that is arranged opposite to the source alignment flange.

5. A rotational angle measurement system, comprising:
- the rotational angle measurement device (10) according to any one of the preceding claims; and
- a processing device (70) configured to determine a rotational angle of a rotatable X-ray collimator based on an electrical signal produced by the rotational angle measurement device.

6. The rotational angle measurement system according to claim 5,
wherein the processing device is further configured to perform a calibration of the rotational angle measurement device.

7. The rotational angle measurement system according to claim 6,
wherein the processing device is configured to perform a multi-point calibration of the rotational angle measurement device.

8. The rotational angle measurement system according to claim 7,
wherein the multi-point calibration comprises a seven-point calibration, wherein the seven-point calibration comprises the following angular steps: -45°, -30°, -15°, 0°, 15° , 30°, and 45°.

9. An X-ray collimator (100), comprising:
- the rotational angle measurement device according to any one of claims 1 to 4; or
- the rotational angle measurement system according to any one of claims 5 to 8.

10. A rotational angle measurement method (300) for measuring a rotational angle of a rotatable X-ray collimator, the method comprising:
- controlling (310) the rotatable X-ray collimator to adjust a rotational position;
- obtaining (320) an electrical signal from a rotational angle measurement device according to any one of claims 1 to 4; and
- determining (330) a rotational angle of the rotatable X-ray collimator based on the electrical signal.

11. The rotational angle measurement method according to claim 10, further comprising:
- performing a calibration of the rotational angle measurement device.

12. The rotational angle measurement method according to claim 11,
wherein the step of performing a calibration of the rotational angle measurement device comprises:
- performing a multi-point calibration of the rotational angle measurement device.

13. The rotational angle measurement method according to claim 11,
wherein the multi-point calibration comprises a seven-point calibration, wherein the seven-point calibration comprises the following angular steps: -45°, -30°, -15°, 0°, 15° , 30°, and 45°.

14. A computer program element comprising instructions which, when the program is executed by a processing device (70) of a rotational angle measurement system of any one of claims 5 to 8, cause the processing device (70) to induce a performing of the steps of the method of any one of claims 10 to 13.

15. A computer-readable storage medium having stored thereon the computer program product of claim 14.

## Patentansprüche

1. Drehwinkelmessvorrichtung (10) zum Messen eines Drehwinkels eines drehbaren Röntgenkollimators, wobei die Vorrichtung umfasst:
- einen Kolben (12); und
- einen Widerstandsstreifen (14);
wobei der Widerstandsstreifen ein längliches Band umfasst, welches an einem ersten Abschnitt des drehbaren Röntgenkollimators befestigt ist, und eine Längserstreckung des länglichen Bandes so angeordnet ist, dass sie um die Drehachse des drehbaren Röntgenkollimators gekrümmt ist;
wobei der Kolben an einem zweiten Abschnitt des Röntgenkollimators befestigt ist, wobei der erste Abschnitt und der zweite Abschnitt so angeordnet sind, dass sie sich während einer Drehung des Röntgenkollimators relativ zueinander drehen; und
wobei der Kolben so angeordnet ist, dass er mit dem länglichen Streifen in Kontakt steht, sodass eine Drehung des drehbaren Röntgenkollimators den länglichen Streifen relativ zu dem Kolben bewegt, um ein elektrisches Signal zu erzeugen, welches einen Drehwinkel des drehbaren Röntgenkollimators anzeigt.

2. Drehwinkelmessvorrichtung nach Anspruch 1,
wobei der Kolben eines der Folgenden umfasst:
- einen federbelasteten Kolben; und
- einen druckbelasteten Kolben.

3. Drehwinkelmessvorrichtung nach Anspruch 1 oder 2,
wobei der zweite Abschnitt einen Quellenausrichtungsflansch (40) umfasst, welcher dazu angeordnet ist, den Röntgenkollimator mit der Röntgenquelle zu verbinden.

4. Drehwinkelmessvorrichtung nach Anspruch 3,
wobei der erste Abschnitt eine Streifenmontagehalterung (60) umfasst, welche gegenüber dem Quellenausrichtungsflansch angeordnet ist.

5. Drehwinkelmesssystem, umfassend:
- die Drehwinkelmessvorrichtung (10) nach einem der vorstehenden Ansprüche; und
- eine Verarbeitungsvorrichtung (70), welche dazu konfiguriert ist, einen Drehwinkel eines drehbaren Röntgenkollimators basierend auf einem von der Drehwinkelmessvorrichtung produzierten elektrischen Signal zu bestimmen.

6. Drehwinkelmesssystem nach Anspruch 5,
wobei die Verarbeitungsvorrichtung weiter dazu konfiguriert ist, eine Kalibrierung der Drehwinkelmessvorrichtung durchzuführen.

7. Drehwinkelmesssystem nach Anspruch 6,
wobei die Verarbeitungsvorrichtung dazu konfiguriert ist, eine Mehrpunktkalibrierung der Drehwinkelmessvorrichtung durchzuführen.

8. Drehwinkelmesssystem nach Anspruch 7,
wobei die Mehrpunktkalibrierung eine Siebenpunktkalibrierung umfasst, wobei die Siebenpunktkalibrierung die folgenden Winkelschritte umfasst: -45°, -30°, -15°, 0°, 15°, 30° und 45°.

9. Röntgenkollimator (100), umfassend:
- die Drehwinkelmessvorrichtung nach einem der Ansprüche 1 bis 4; oder
- das Drehwinkelmesssystem nach einem der Ansprüche 5 bis 8.

10. Drehwinkelmessverfahren (300) zum Messen eines Drehwinkels eines drehbaren Röntgenkollimators, wobei das Verfahren umfasst:
- Steuern (310) des drehbaren Röntgenkollimators zum Einstellen einer Drehposition;
- Erhalten (320) eines elektrischen Signals von einer Drehwinkelmessvorrichtung nach einem der Ansprüche 1 bis 4; und
- Bestimmen (330) eines Drehwinkels des drehbaren Röntgenkollimators basierend auf dem elektrischen Signal.

11. Drehwinkelmessverfahren nach Anspruch 10, weiter umfassend:
- Durchführen einer Kalibrierung der Drehwinkelmessvorrichtung.

12. Drehwinkelmessverfahren nach Anspruch 11,
wobei der Schritt des Durchführens einer Kalibrierung der Drehwinkelmessvorrichtung umfasst:
- Durchführen einer Mehrpunktkalibrierung der Drehwinkelmessvorrichtung.

13. Drehwinkelmessverfahren nach Anspruch 11,
wobei die Mehrpunktkalibrierung eine Siebenpunktkalibrierung umfasst, wobei die Siebenpunktkalibrierung die folgenden Winkelschritte umfasst: -45°, -30°, -15°, 0°, 15°, 30° und 45°.

14. Computerprogrammelement, welches Anweisungen umfasst, welche, wenn das Programm von einer Verarbeitungsvorrichtung (70) eines Drehwinkelmesssystems nach einem der Ansprüche 5 bis 8 ausgeführt wird, die Verarbeitungsvorrichtung (70) dazu veranlassen, die Durchführung der Schritte des Verfahrens nach einem der Ansprüche 10 bis 13 zu bewirken.

15. Computerlesbares Speichermedium, auf dem das Computerprogrammprodukt nach Anspruch 14 gespeichert ist.

## Revendications

1. Dispositif de mesure d'angle de rotation (10) pour mesurer un angle de rotation d'un collimateur à rayons X rotatif, le dispositif comprenant :
- un poussoir (12) ; et
- une bande résistive (14) ;
dans lequel la bande résistive comprend une bande allongée qui est attachée à une première portion du collimateur à rayons X rotatif, et une étendue longitudinale de la bande allongée est agencée pour être courbée autour de l'axe de rotation du collimateur à rayons X rotatif ;
dans lequel le poussoir est attaché à une seconde portion du collimateur à rayons X, dans lequel la première portion et la seconde portion sont agencées pour tourner l'une par rapport à l'autre pendant une rotation du collimateur à rayons X ; et
dans lequel le poussoir est agencé pour être en contact avec la bande allongée de sorte qu'une rotation du collimateur à rayons X rotatif déplace la bande allongée par rapport au poussoir pour produire un signal électrique indicatif d'un angle de rotation du collimateur à rayons X rotatif.

2. Dispositif de mesure d'angle de rotation selon la revendication 1,
dans lequel le poussoir comprend un parmi :
- un poussoir à ressort ; et
- un poussoir sous pression.

3. Dispositif de mesure d'angle de rotation selon la revendication 1 ou 2,
dans lequel la seconde portion comprend une bride d'alignement de source (40) qui est agencée pour coupler le collimateur à rayons X à la source de rayons X.

4. Dispositif de mesure d'angle de rotation selon la revendication 3,
dans lequel la première portion comprend un support de montage de bande (60) qui est agencé à l'opposé de la bride d'alignement de source.

5. Système de mesure d'angle de rotation comprenant :
- le dispositif de mesure d'angle de rotation (10) selon l'une quelconque des revendications précédentes ; et
- un dispositif de traitement (70) configuré pour déterminer un angle de rotation d'un collimateur à rayons X rotatif sur la base d'un signal électrique produit par le dispositif de mesure d'angle de rotation.

6. Système de mesure d'angle de rotation selon la revendication 5,
dans lequel le dispositif de traitement est en outre configuré pour effectuer un étalonnage du dispositif de mesure d'angle de rotation.

7. Système de mesure d'angle de rotation selon la revendication 6,
dans lequel le dispositif de traitement est configuré pour effectuer un étalonnage multipoint du dispositif de mesure d'angle de rotation.

8. Système de mesure d'angle de rotation selon la revendication 7,
dans lequel l'étalonnage multipoint comprend un étalonnage à sept points, dans lequel l'étalonnage à sept points comprend les étapes angulaires suivantes : -45°, -30°, - 15°, 0°, 15°, 30° et 45°.

9. Collimateur à rayons X (100) comprenant :
- le dispositif de mesure d'angle de rotation selon l'une quelconque des revendications 1 à 4 ; ou
- le système de mesure d'angle de rotation selon l'une quelconque des revendications 5 à 8.

10. Procédé de mesure d'angle de rotation (300) pour mesurer un angle de rotation d'un collimateur à rayons X rotatif, le procédé comprenant :
- la commande (310) du collimateur à rayons X rotatif pour ajuster une position de rotation ;
- l'obtention (320) d'un signal électrique d'un dispositif de mesure d'angle de rotation selon l'une quelconque des revendications 1 à 4 ; et
- la détermination (330) d'un angle de rotation du collimateur à rayons X rotatif sur la base du signal électrique.

11. Dispositif de mesure d'angle de rotation selon la revendication 10, comprenant en outre :
- la réalisation d'un étalonnage du dispositif de mesure d'angle de rotation.

12. Procédé de mesure d'angle de rotation selon la revendication 11,
dans lequel l'étape de mise en œuvre d'un étalonnage du dispositif de mesure de l'angle de rotation comprend :
- la réalisation d'un étalonnage multipoint du dispositif de mesure d'angle de rotation.

13. Procédé de mesure d'angle de rotation selon la revendication 11,
dans lequel l'étalonnage multipoint comprend un étalonnage à sept points, dans lequel l'étalonnage à sept points comprend les étapes angulaires suivantes : -45°, -30°, - 15°, 0°, 15°, 30° et 45°.

14. Élément de programme informatique comprenant des instructions qui, lorsque le programme est exécuté par un dispositif de traitement (70) d'un système de mesure d'angle de rotation selon l'une quelconque des revendications 5 à 8, amènent le dispositif de traitement (70) à induire une mise en œuvre des étapes du procédé selon l'une quelconque des revendications 10 à 13.

15. Support de stockage lisible par ordinateur sur lequel est stocké le produit de programme informatique selon la revendication 14.
